# EUROPEAN PATENT SPECIFICATION

(11) **EP 1 466 572 B1**
(45) Date of publication and mention of the grant of the patent: **23.01.2008**
(21) Application number: 04004678.1
(22) Date of filing: 01.03.2004
(51) Int. Cl.: A61F 2/46, A61B 17/88, B01F 13/00, B05C 17/005, A61J 1/00

(54) **Device for packaging, mixing and applying bone cement**
Vorrichtung zum Verpacken, Mischen und Applizieren von Knochenzement
Dispositif pour conditionner, mélanger et appliquer du ciment à os

(30) Priority: 04.03.2003 IT MO20030057
(43) Date of publication of application: 13.10.2004
(73) Proprietor: Sidam di Azzolini Graziano E C. S.a.s., 41030 San Giacomo Roncole - Mirandola, (Prov. of Modena) (IT)
(72) Inventor: Azzolini, Graziano, 41032 Cavezzo (Prov. of Modena) (IT)
(74) Representative: Brogi, Graziano

(56) References cited:
- EP-A- 0 266 058
- EP-A- 0 525 480
- WO-A-98/57734
- US-A- 4 306 554
- US-A- 4 463 875
- US-A- 4 551 135
- US-A- 5 697 918
- US-A- 5 951 160
- US-A1- 2001 037 091
- US-A1- 2002 101 785
- US-A1- 2002 118 595

## Description

The present invention relates to a device for packaging, mixing and applying bone cement.

The expression "bone cement" is used to designate a biocompatible material that is normally constituted by a resin and a catalyst and is used in various fields of medicine, such as for example orthopedics, operative radiology and surgery, for positioning prostheses, repairing bone fractures caused for example by osteoporosis, preventing and treating vertebral fractures caused by tumor lesions (metastasis, myeloma, angiomas) and alleviate the consequent pain.

For example, the technique of percutaneous vertebroplasty is known; it consists in producing a hole, under local anesthesia and with visual monitoring performed radiologically and/or by CAT scan, in the affected vertebra, by means of an appropriately provided cannula the opening of which is temporarily closed by a stylet, in extracting the stylet from the cannula thus inserted in the vertebra, and in injecting bone cement into the opening by using an appropriately provided syringe or gun.

The injected bone cement hardens inside the vertebra to repair any lesions thereof, reconstruct it, reinforce it or stabilize any tumors.

Bone cements currently in use are mixtures of at least two components: typical bone cements are acrylic resins, such as PMMA, which are obtained by mixing two precursors, of which one is generally in the liquid state (catalyst) and the other is generally in powder form; the polymerization reaction produces the cement compound.

The two components of the cement are individually packaged in separate containers, such as for example vials, bottles, sachets, pouches or the like, and are mixed together only when the cement is to be used, in order to prevent it from hardening even before it is injected or applied.

Currently known devices for mixing the two components substantially consist of a bowl and of a spatula; when the cement is to be used, a health or medical worker opens the containers of the two components and pours their content into the bowl, inside which, by using the spatula, he mixes them until a sticky fluid or paste is obtained.

The worker then takes from the bowl a selected dose of paste or fluid by aspirating it with the syringe or gun, with which it is then injected for example into a vertebroplasty cannula that is already inserted in a patient.

Mixing devices are also known which are constituted by a tray provided with a removable closure lid, with which an agitator blade is rotatably associated; said blade can be inserted in the tray and actuated manually so as to rotate about its own axis by way of a handle that protrudes outside said lid.

The lid is further provided with filters for absorbing the fumes and vapors that are generally released during preparation of bone cements.

When the cement is to be used, a worker opens the containers of the two components, pours their content into the open tray, and after closing the tray with the lid acts on the handle in order to turn the agitator blade and thus mix the two components until a sticky fluid or paste is obtained.

The operator then removes the lid in order to reopen the tray and remove from it a selected dose of paste or fluid, aspirating it with the syringe or gun.

These known devices are not free from drawbacks, including the fact that they expose medical and health workers directly to the fumes and vapors released during the mixing of the two components of the bone cement, which are toxic in addition to having a bad odor.

Exposure to the fumes and vapors is not avoided even in the case of devices constituted by a tray that can be closed by means of a lid provided with filters; mixing of the two components and removal of the prepared fluid or paste in fact occur while the tray is open.

The fumes and vapors might be removed by preparing the cement under extractor hoods, which, however, are not installed in the operating rooms or radiology rooms inside which the cement is often prepared to be used immediately, such as for example in cases of vertebroplasty.

Another drawback of known devices is that removing the prepared fluid or paste from the bowl or tray by means of a syringe or gun is awkward and increases the time required for its application.

Another drawback of known devices is that in view of possible spillage of the two components of the cement and of the amounts of fluid or paste that remain stuck to the walls of the bowl or tray, they usually entail preparing amounts of cement that exceed the doses actually to be applied and therefore entail discarding the unused excess, with a consequent onerous waste of materials.

In particular, document US 2002/0101785 A1 discloses an apparatus, for mixing two components forming a cement bone, which comprises two syringes in which there are contained respectively a first basic component, in form of powder, and a second basic component, in liquid form, for a bone cement.

The apparatus further comprises a specific mixing mechanism where the two syringes are initially positioned, in such a way to be connected one to the other through respective ports, and where they are successively moved back and forth, so as to cause mixing of the two basic component at the inside of one syringe, and transferring of the mixture thus obtained from a syringe to the other, more times, until the mixing is complete and the bone cement formed.

Between the prior art documents, which disclose solutions based on the use of two containers containing in separate form the two basic components intended to be mixed for forming a bone cement, it is also mentioned US 5,951,160.

More in detail, this document discloses an apparatus which comprises two distinct containers, specifically constituted by a syringe and a flexible bag, in each of which there is packaged in single form a respective basic component for a bone cement, wherein the two containers are successively connected together through the interposition of a closure mechanism for transferring one of the components from the syringe to the flexible bag, so as to mix the two basic components and thereby form the bone cement

It is also mentioned document US 2002/0118595 A1 which discloses a movable ball of metal which is immerged within the fluid mixture, filling a vial, formed by two basic components for a bone cement, whereby, when the vial is manually shaken up and down, the ball moves through the fluid mixture so as to facilitate mixing of the two basic components.

The solution disclosed by this last document is far from and considerably less practical to use in comparison with the solutions which comprise and are based on two separate containers, provided for being reciprocally connected and each containing a respective basic component for forming the bone cement.

However also the solutions proposed by the other two before mentioned documents are not completely exempt from drawbacks, whereby they would require to be further improved.

Therefore, in view of the above documents, one the objects of the present invention can be regarded as that of providing a device, for preparing bone cement from two basic components, in particular a liquid component and a powder component, which is more practical and easier to use than the solutions at present known and used, including even those which provide a kit of two distinct and separate containers, for the two basic components, intended to be connected for mixing them and thereby forming the bone cement.

The aim of the present invention is to eliminate the above-noted drawbacks of known devices, by providing a device for packaging, mixing and applying bone cement that allows to protect medical and health workers from exposure to malodorous and toxic fumes and vapors, to prepare the cement simply, easily and rapidly, and to contain the waste of materials.

Within this aim, an object of the present invention is to provide a device that is simple, relatively easy to provide in practice, safe in use, effective in operation, and has a relatively low cost.

This aim and this and other objects that will become better apparent hereinafter are achieved by the present device for packaging, mixing and applying bone cement obtainable from at least one first component and one second component, characterized in that it comprises a first container, in which said first component is packaged hermetically and which is provided with an opening that is associated with first temporary closure means; a second substantially cylindrical container, which is provided with an outlet that is associated with second temporary closure means; and piston means, which are inserted so that they can slide hermetically within said second container and can be actuated from outside with a rectilinear motion; said second component being packaged between said outlet and said piston means; said opening and said outlet being temporarily mutually associable, and said piston means being suitable to push said second component from said second container to said first container for mixing with said first component and to aspirate the cement thus formed from said first container into said second container.

Further characteristics and advantages of the present invention will become better apparent from the following detailed description of a preferred but not exclusive embodiment of a device for packaging, mixing and applying bone cement, illustrated by way of non-limiting example in the accompanying drawings, wherein:
Figure 1 is a schematic perspective view of a device for packaging, mixing and applying bone cement according to the invention in the configuration for packaging the two components of the cement;

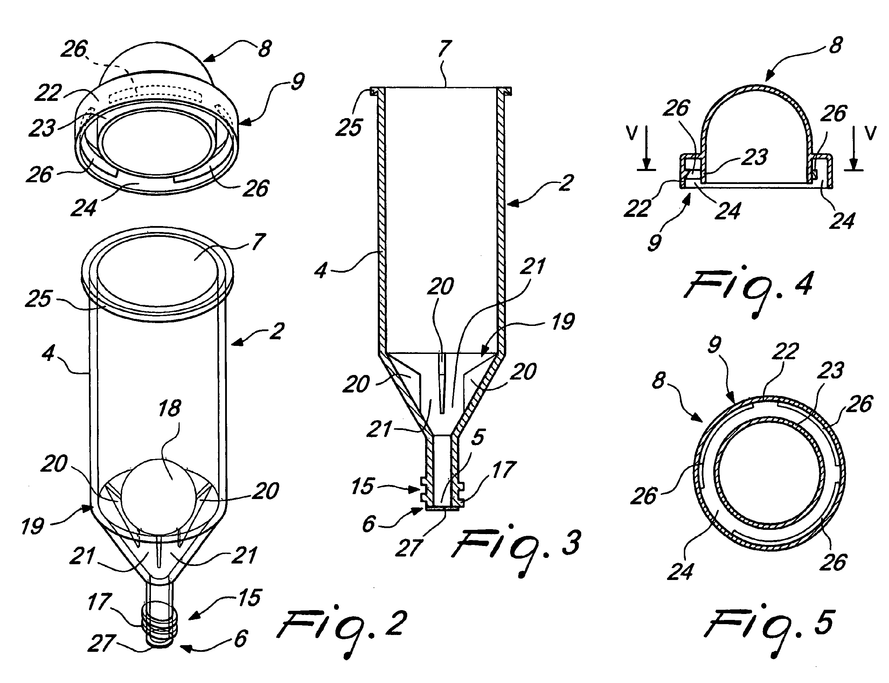

Figure 2 is a partially exploded schematic perspective view of the first container of the device according to the invention;
Figure 3 is a schematic sectional view of a detail of the first container of the device according to the invention;
Figure 4 is a schematic sectional view of another detail of the first container of the device according to the invention;
Figure 5 is a sectional view, taken along the line V-V, of the detail of Figure 4;
Figure 6 is a schematic perspective view of the device according to the invention in the configuration for mixing the two components for preparing the bone cement;
Figure 7 is a schematic perspective view of the second container of the device according to the invention in the configuration for applying the prepared bone cement.

With reference to the figures, the reference numeral 1 generally designates a device for packaging, mixing and applying bone cement.

The expression "bone cement" is used to designate a biocompatible material that is normally constituted by a resin and a catalyst and is used in various fields of medicine, such as for example orthopedics, operative radiology and surgery, for positioning prostheses, repairing bone fractures caused for example by osteoporosis, preventing and treating vertebral fractures caused by tumor lesions (metastasis, myelomas, angiomas), alleviating the consequent pain.

A bone cement is usually obtained by mixing two components or precursors: a first component A, which is generally in the form of a powder or the like, and a second component B, which is generally in the fluid, liquid, pasty or gel state.

Merely by way of example, currently used bone cements include acrylic resins, including in particular PMMA; the two components are mixed shortly before the cement is applied, in order to prevent it from becoming unusable due to the extremely short setting and hardening times.

The device 1 comprises a first container 2, in which the first component A is packaged hermetically, and a second container 3, in which the second component B is packaged hermetically.

The first container 2 acts as a reservoir for the first component A and as a mixing chamber for the first component A and the second component B in order to obtain the cement C; the second container 3 acts as a reservoir for the second component B and as an applicator for the cement C.

The first container 2 is constituted by a substantially cylindrical body 4, which is open at its opposite ends; an opening 5 is formed at one end for the inflow of the second component B and for the outflow of the cement C and is associated with first temporary closure means 6, while at the opposite end a filling opening 7 is provided, through which the first component A is inserted in the body 4 during packaging, said opening being associated with a closure element such as a cap 8.

Coupling means 9 of the interlocking type, suitable to ensure hermetic sealing of the first component A, are formed between the cap 8 and the filling opening 7.

Advantageously, the second container 3 is a syringe or gun and comprises a substantially cylindrical cannula 10 that is open at its opposite ends; an outlet 11 is formed at one end for the outflow of the second component B and for aspirating and dispensing the cement C, and is associated with second temporary closure means 12; at the opposite end piston means 13 are instead provided, which can be inserted slidingly and hermetically in the cannula 10 and can be actuated from outside with a reciprocating rectilinear motion.

The second component B is packaged between the outlet 11 and the piston means 13.

The piston means 13 are actuated by means of a stem 14, which is of the removable and reusable type, such as the one shown in the figures, or is formed monolithically with said piston means.

There are also means for temporary connection 15 between the opening 5 and the outlet 11, which in the case shown in the figures are constituted by a male connector 16 and a corresponding female connector 17 of the Luer-Lok type, formed respectively at the outlet 11 and at the opening 5; however, other embodiments of the temporary connection means 15 are also possible.

By way of the temporary connection means 15, the opening 5 and the outlet 11 are temporarily mutually associated, after or simultaneously with the removal of the first and second temporary closure means 6 and 12 respectively, in order to connect the first container 2 and the second container 3 for preparing the cement C.

Movable agitator means are inserted within the first container 2 and are constituted for example by a ball 18 that can move freely and whose specific gravity is greater than that of the first component A and of the cement C.

Advantageously, means 19 for retaining the ball 18 or other agitator means are formed inside the body 4 of the first container 2 at a preset distance from the opening 5 in order to prevent its blockage.

The retention means 19 comprise for example a plurality of longitudinal, radial and centripetal ribs or wings 20, which are distributed along the perimeter of the inside wall of the body 4 proximate to the opening 5.

The ribs 20 retain the ball 18 at a preset distance from the opening 5, thus preventing it from being blocked and leaving open passages 21 for the flow of the two components A and B and of the cement C.

As an alternative, the retention means can be constituted for example by one or more raised portions, for example shaped like a circular arc, which protrude centripetally on the inside walls of the body 4 proximate to the opening 5 and are mutually spaced so as to retain the ball 18 and leave free passages for the two components A and B and for the cement C.

Proximate to the outside perimeter of the cap 8 there are two folded and mutually substantially coaxial lips, namely an outer lip 22 and an inner lip 23, between which an annular ring 24 is formed; the edge 25 that delimits the filling opening 7 of the body 4 can be inserted in said ring with a snap action.

The coupling means 9 comprise one or more circular arc-like teeth 26, which are distributed with a constant spacing along the outer lip 22 and are directed toward the inside of the annular ring 24 in order to engage by snap action the edge 25, which is advantageously beveled.

Conveniently, the cap 8 is shaped like a dome or the like.

In the illustrated embodiment, the first temporary closure means 6 are constituted by a tearable membrane 27, a film or the like, the edges of which, once torn, continue to adhere to the rim of the opening 5 in order to prevent fragments thereof from being mixed in with the cement C. However, other embodiments of the first temporary closure means 6 are also possible and might be constituted for example by a membrane, a film of the type that can be removed by peeling, a cap or the like of the removable type, or a pad or the like that is associated with the opening 5 along lines that are at least partially of the preweakened type so as to remain attached in at least one point to the rim of the opening 5 so as to prevent its accidental introduction in the body 4.

The second temporary closure means 12 are instead constituted by a stopper 28 or the like that is detachably associated with the outlet 11.

However, further embodiments of the second temporary closure means 12 are also possible and might be constituted for example by a membrane, a film, a tearable or peelable film, or by a pad or the like that is associated with the outlet 11 along lines that are at least partially of the preweakened type.

Conveniently, moreover, the first temporary closure means 6 can be constituted by a check valve of the ball type, which is retained in the configuration for closing the opening 5 by elastic means; the valve would be opened for example by the male connector 16 when the outlet 11 is associated with the opening 5 and closed when the outlet 11 and the opening 5 are mutually disconnected.

Conveniently, the device 1 is made of plastics and is of the disposable type, while the ball 18 is made of glass, metal, plastics or the like.

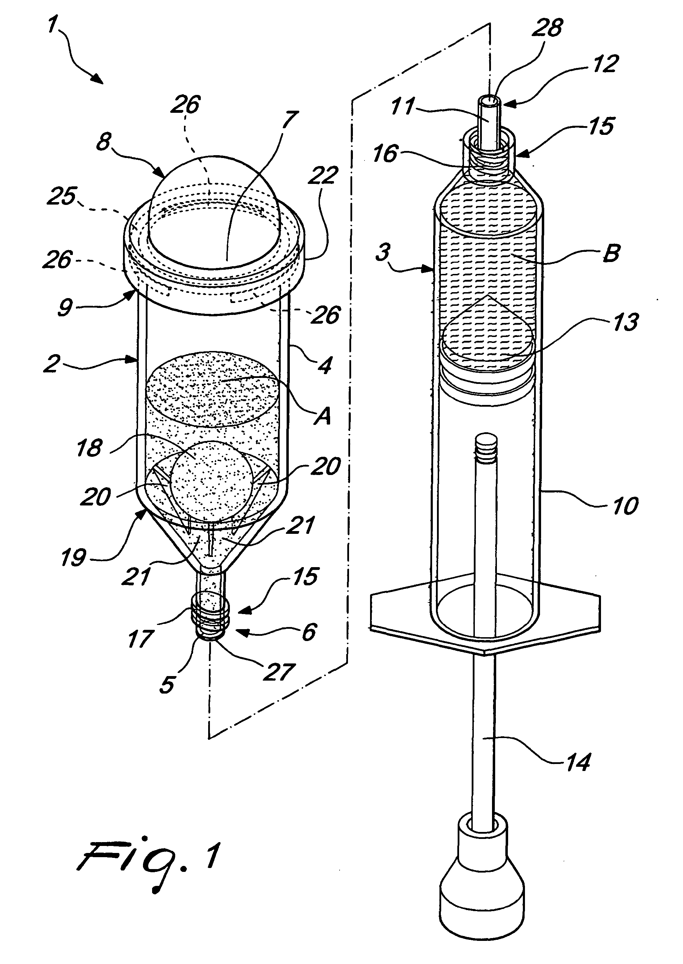

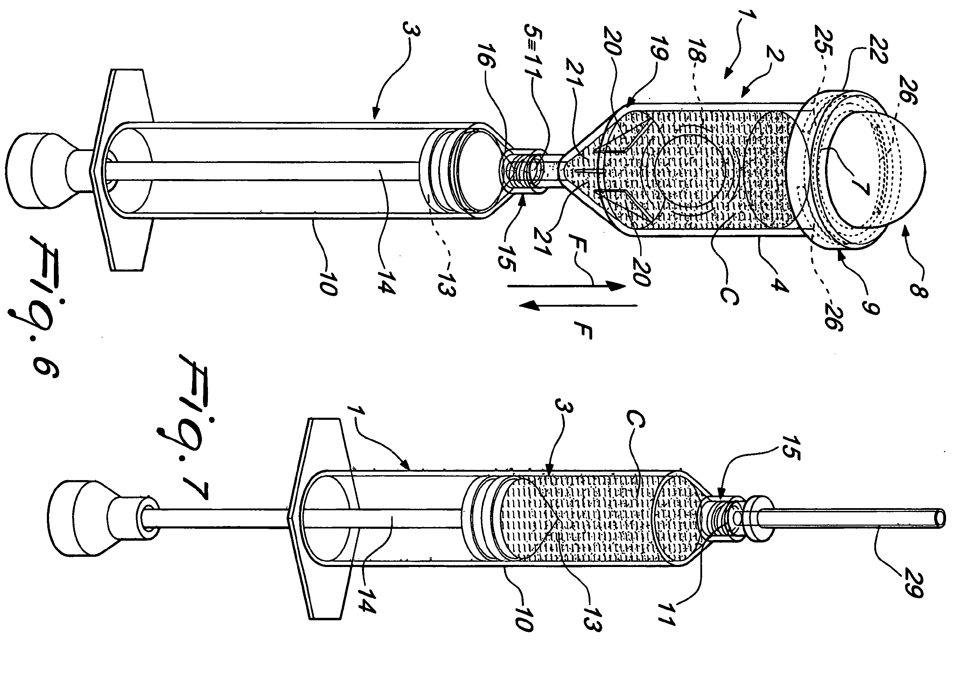

The operation of the invention is as follows. The device 1 assumes a configuration for packaging the first component A and the second component B so that they are mutually separate (Figure 1), a configuration for mixing the first component A and the second component B in order to prepare the cement C (Figure 6), and a configuration for applying the cement C thus prepared (Figure 7).

In the packaging configuration, the first component A is packaged hermetically inside the first container 2, the opening 5 is closed by the membrane 27, and the filling opening 7, through which the body 4 has been filled, is closed hermetically by the cap 8.

The second component B is instead packaged hermetically inside the second container 3, the outlet 11 is closed by the stopper 28 and the piston means 13 are in the fixed position.

When it is necessary to apply the bone cement, the device 1 acts as a mixer and as an applicator of said cement.

In order to pass from the packaging configuration to the mixing configuration, it is sufficient to remove the stopper 28 and associate the outlet 11 with the opening 5 by screwing the male connector 16 into the female connector 17; during this connection step, the membrane 27 is torn in order to connect the cannula 10 to the body 4; the membrane 27, however, remains attached to the rim of the opening 5, so that it cannot mix with the cement being formed.

By way of the stem 14, the piston means 13 are pushed toward the outlet 11 in order to transfer the second component B from the cannula 10 into the body 4, inside which it mixes with the first component A.

By shaking the first container 2 and the second container 3, even manually, in this configuration in which they are thus monolithically associated, with movements that are shown schematically by the arrows F, mixing of the first component A and of the second component B to form the cement C is facilitated.

Mixing is assisted by the ball 18 or by other agitator means, which during shaking, thanks to its higher specific gravity, stirs the cement being formed, and by the shape of the cap 8, which by being rounded avoids accumulations of the two separate components.

After reaching the sufficient degree of mixing, the second container 3 of the device 1 can be used to apply the cement C.

In order to assume the application configuration, keeping the outlet 11 associated with the opening 5 and therefore keeping the first container 2 connected to the second container 3, one acts on the stem 14 so as to move the piston means 13 away from the outlet 11 and thus aspirate the cement C from the body 4 inside the cannula 10.

Once the selected dose of cement C has been aspirated, by unscrewing the male connector 16 from the female connector 17 the second container 3 is disengaged from the first container 2.

If the first temporary closure means 6 are constituted by a check valve of the ball type, when the second container 3 is disengaged from the first container 2 the opening 5 is closed automatically, avoiding any leakage or release of fumes from the body 4.

The second container 3 (syringe) can thus be used to apply the dose of cement C aspirated therein after screwing onto the male connector 16 an injector, such as a tube 29, provided with a corresponding female connector.

In practice it has been found that the described invention achieves the intended aim and objects.

The device according to the invention in fact prevents medical or health workers from being exposed to the fumes and vapors released during the mixing of the components of the cement, since said mixing always occurs in a closed environment.

Moreover, the device according to the invention allows to package and mix predefined doses of cement, to limit the waste and costs of materials, and to prepare the cement simply, easily and rapidly.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device (1) for packaging, mixing and applying bone cement (C) obtainable from at least one first component (A) and one second component (B), comprising a first container (2), in which said first component (A) is packaged hermetically and which is provided with an opening (5) that is associated with first temporary closure means (6); a second substantially cylindrical container (3), which is provided with an outlet (11) that is associated with second temporary closure means (12); and piston means (13), which are inserted so that they can slide hermetically within said second container (3) and can be actuated from outside with a rectilinear motion; said second component (B) being packaged between said outlet (11) and said piston means (13); said opening (5) and said outlet (11) being temporarily mutually associable, and said piston means (13) being suitable to push said second component (B) from said second container (3) to said first container (2) for mixing with said first component (A) and to aspirate the cement (C) thus formed from said first container (2) into said second container (3), said device being **characterized in that** said first container (2) further comprises agitator means (18) which are arranged for moving inside it so as to mix together said first (A) and said second component (B) when said first container (2) is shaken.

2. The device according to claim 1, **characterized in that** said second component (B) is in fluid, paste, or gel state, and **in that** said second container (3) is a syringe, or a gun.

3. The device according to one or more of the preceding claims, **characterized in that** said first component (A) is in the form of a powder.

4. The device according to one or more of the preceding claims, **characterized in that** it comprises means for temporary connection (15, 16, 17) between said opening (5) and said outlet (11).

5. The device according to claim 4, **characterized in that** said temporary connection means (15) comprise a male connector (16) and a corresponding female connector (17) of the Luer-Lok type, formed respectively at said outlet (11) and at said opening (5) or vice versa.

6. The device according to one or more of the preceding claims, **characterized in that** said agitator means comprise a ball (18) that is inserted loosely in said first container (2), the specific gravity of said ball (16) being higher than that of said first component (A) and of said cement (C).

7. The device according to one or more of the preceding claims, **characterized in that** said first container (2) comprises means (20) for retaining said agitator means (18) at a preset distance from said opening (5) in order to prevent its blockage.

8. The device according to claim 7, **characterized in that** said retaining means (20) comprise at least one protrusion that is formed so that it protrudes centripetally proximate to said opening (5).

9. The device according to claim 7, **characterized in that** said retaining means comprise a plurality of wings or ribs (20), which are longitudinal, radial and centripetal and are distributed along the inside perimeter of said first container (2).

10. The device according to one or more of the preceding claims, **characterized in that** said first container (2) comprises a filling opening (7) which is associated with a closure element (8).

11. The device according to claim 10, **characterized in that** said closure element is constituted by a cap (8), means for the interlocking coupling (9) of said cap (8) to said first container (2) being provided.

12. The device according to claim 11, **characterized in that** said interlocking coupling means (9) comprise at least one tooth (26) that is formed along the outside perimeter of said cap (8) and can engage by snap action the edge of said filling opening (7).

13. The device according to claim 12, **characterized in that** the teeth (26) are arranged in an arc-like configuration along said outside perimeter of the cap (8), along which they are distributed with a constant spacing.

14. The device according to one or more of the 12 to 13, **characterized in that** said cap (8) comprises two annular lips (22, 23) that are substantially coaxial and folded, are formed proximate to said outside perimeter, and between which an annular ring (24) is formed, the rim of said filling opening (7) being insertable in said annular ring (24).

15. The device according to claim 14, **characterized in that** said teeth (26) are formed along at least one of said lips and face the inside of said annular ring (24).

16. The device according to one or more of the claims 10 to 15, **characterized in that** said closure element (8) is substantially shaped like a dome (8).

17. The device according to one or more of the preceding claims, **characterized in that** said first (6) or second temporary closure means (12) comprise a stopper.

18. The device according to one or more of the claims 1 to 16, **characterized in that** said first (6) or second temporary closure means (12) comprise a membrane, or a film of the tearable, removable or other type.

19. The device according to one or more of the claims 1 to 16, **characterized in that** said first (6) or second temporary closure means (12) comprise a pad that is respectively associated with said opening (5) or with said outlet (11) along lines that are at least partially of the preweakened type.

20. The device according to one or more of the claims 1 to 16, **characterized in that** said first (6) or second temporary closure means (12) comprise a check valve of the ball type.

## Patentansprüche

1. Vorrichtung (1) zum Verpacken, Mischen und Applizieren von Knochenzement (C), den man aus mindestens einer ersten Komponente (A) und einer zweiten Komponente (B) erhält, mit einem ersten Gefäß (2), in dem die erste Komponente (A) hermetisch verpackt ist, und das eine Öffnung (5) aufweist, welche mit ersten, temporären verschlusselementen (6) verbunden ist; einem zweiten, im wesentlichen zylindrischen Gefäß (3), das einen Austritt (11) besitzt, der mit sekundären, temporären Verschlusselementen (12) verbunden ist; und Kolbenelementen (13), die eingeschoben werden, so dass sie in dem zweiten Gefäß (3) hermetisch verschoben werden können, und mit einer geradlinigen Bewegung von außen betätigt werden können, wobei die zweite Komponente (B) zwischen dem Austritt (11) und den Kolbenelementen (13) verpackt ist, und die Öffnung (5) und der Austritt (11) vorübergehend miteinander verbunden werden können, und die zweite Komponente (B) zum Mischen mit der ersten Komponente (A) und um den so hergestellten Zement (C) von dem ersten Gefäß (2) in das zweite Gefäß (3) anzusaugen, mit den Kolbenelementen vom zweiten Gefäß (3) zum ersten Gefäß (2) verschoben werden kann, wobei die Vorrichtung **dadurch gekennzeichnet ist, dass** das erste Gefäß (2) weiterhin ein Rührwerk (18) besitzt, das für eine Bewegung im Inneren angeordnet ist, um die erste (A) Komponente und die zweite Komponente (B) miteinander zu vermischen, wenn das erste Gefäß (2) geschüttelt wird.

2. Vorrichtung nach Anspruch 1, **dadurch gekennzeichnet, dass** es sich bei der zweiten Komponente (B) um eine Flüssigkeit, Paste oder Gel handelt, und dass es sich bei dem zweiten Gefäß (3) um eine Spritze oder Pistole handelt.

3. Vorrichtung nach einem oder mehreren der vorhergehenden Ansprüche, **dadurch gekennzeichnet, dass** die erste Komponente (A) Pulverform hat.

4. Vorrichtung nach einem oder mehreren der vorhergehenden. Ansprüche, **dadurch gekennzeichnet, dass** sie Elemente zur vorübergehenden Verbindung (15, 16, 17) zwischen der Öffnung (5) und dem Austritt (11) besitzt.

5. Vorrichtung nach Anspruch 4, **dadurch gekennzeichnet, dass** die vorübergehenden Verbindungselemente (15) einen Luer-Lok Stecker (16) und eine entsprechende Luer Lok-Buchse (17) besitzen, die jeweils am Austritt (11) und an der Öffnung (5) oder umgekehrt gebildet werden.

6. Vorrichtung nach einem oder mehreren der vorhergehenden Ansprüche, **dadurch gekennzeichnet, dass** das Rührwerk eine Kugel (18) aufweist, die locker in das erste Gefäß (2) eingeschoben ist, wobei das spezifische Gewicht der Kugel (16) höher ist als das der ersten Komponente (A) des Zementes (C).

7. Vorrichtung nach einem oder mehreren der vorhergehenden Ansprüche, **dadurch gekennzeichnet, dass** das erste Gefäß (2) Einrichtungen (20) besitzt, um das Rührwerk (18) in einem vorher festgelegten Abstand zu der Öffnung (5) zu halten, um zu verhindern, dass diese versperrt wird.

8. Vorrichtung nach Anspruch 7, **dadurch gekennzeichnet, dass** die Halteelemente (20) mindestens einen Vorsprung aufweisen, der derart ausgebildet ist, dass er zentripetal in der Nähe der Öffnung (5) vorsteht.

9. Vorrichtung nach Anspruch 7, **dadurch gekennzeichnet, dass** die Halteelemente eine Vielzahl von Flügeln oder Rippen (20) aufweisen, die längs, radial und zentripetal und entlang dem inneren Umfang des ersten Gefäßes (2) verteilt sind.

10. Vorrichtung nach einem oder mehreren der vorhergehenden Ansprüche, **dadurch gekennzeichnet, dass** das erste Gefäß (2) eine Füllöffnung (7) besitzt, die mit einem Verschlusselement (8) verbunden ist.

11. Vorrichtung nach Anspruch 10, **dadurch gekennzeichnet, dass** das Verschlusselement aus einer Kappe (8) besteht, wobei Einrichtungen zum Verriegeln der Kupplung (9) der Kappe (8) mit dem ersten Gefäß (2) bereitgestellt werden.

12. Vorrichtung nach Anspruch 11, **dadurch gekennzeichnet, dass** die Verriegelungskupplungselemente (9) mindestens einen Zahn (26) aufweisen, der entlang dem äußeren Umfang der Kappe (8) gebildet ist, und durch Schnappverschluss in die Kante der Füllöffnung (7) eingreifen kann.

13. Vorrichtung nach Anspruch 12, **dadurch gekennzeichnet, dass** die zähne (26) in einer bogenförmigen Konfiguration entlang dem äußeren Umfang der Kappe (8), an dem sie in konstantem Abstand verteilt sind, angeordnet sind.

14. Vorrichtung nach einem oder mehreren der Ansprüche 12 bis 13, **dadurch gekennzeichnet, dass** die Kappe (8) zwei ringförmige Lippen (22, 23) besitzt, die im wesentlichen koaxial und eingeklappt sind, und sich in der Nähe des äußeren Umfanges befinden, und zwischen ihnen ein Ring (24) gebildet wird, wobei der Rand der Füllöffnung (7) in den Ring (24) eingeschoben werden kann.

15. Vorrichtung nach Anspruch 14, **dadurch gekennzeichnet, dass** die Zähne (26) entlang mindestens einer der Lippen gebildet werden, und der Innenseite des Ringes (24) zugewandt sind.

16. Vorrichtung nach einem oder mehreren der Ansprüche 10 bis 15, **dadurch gekennzeichnet, dass** das Verschlusselement (8) im wesentlichen kuppelförming (8) ist.

17. Vorrichtung nach einem oder mehreren der vorhergehenden Ansprüche, **dadurch gekennzeichnet, dass** das erste (6) oder zweite, temporäre Verschlusselement (12) einen Anschlag oder Stopper aufweist.

18. Vorrichtung nach einem oder mehreren der Ansprüche 1 bis 16, **dadurch gekennzeichnet, dass** das erste (6) oder zweite temporäre Verschlusselement (12) eine zerreißbare, abnehmbare oder andersartige Membran oder dünne Schicht aufweist.

19. Vorrichtung nach einem oder mehreren der Ansprüche 1 bis 16, **dadurch gekennzeichnet, dass** das erste (6) oder das zweite Verschlusselement (12) eine unterlage besitzt, die jeweils mit der Öffnung (5) oder mit dem Austritt (11) entlang Linien verbunden ist, die zumindest teilweise vorher abgeschwächt wurden.

20. Vorrichtung nach einem oder mehreren der Ansprüche 1 bis 16, **dadurch gekennzeichnet, dass** das erste (6) oder zweite temporäre Verschlusselement (12) ein Sperrventil vom Typ Kugelventil besitzt.

## Revendications

1. Dispositif (1) pour conditionner, mélanger et appliquer un ciment osseux (C) pouvant être obtenu à partir d'au moins un premier composant (A) et un deuxième composant (B), comprenant un premier récipient (2), dans lequel ledit premier composant (A) est conditionné hermétiquement et qui est muni d'une ouverture (5) qui est associée à des premiers moyens de fermeture temporaire (6) ; un deuxième récipient (3) sensiblement cylindrique qui est muni d'une sortie (11) qui est associée à des deuxièmes moyens de fermeture temporaire (12) ; et des moyens de piston (13) qui sont insérés de telle sorte qu'ils puissent coulisser hermétiquement à l'intérieur dudit deuxième récipient (3) et qu'ils puissent être actionnés depuis l'extérieur avec un mouvement rectiligne ; ledit deuxième composant (B) étant conditionné entre ladite sortie (11) et lesdits moyens de piston (13) ; ladite ouverture (5) et ladite sortie (11) étant temporairement associables mutuellement, et lesdits moyens de piston (13) étant aptes à pousser ledit deuxième composant (B) depuis ledit deuxième récipient (3) en direction dudit premier récipient (2) pour le mélanger audit premier composant (A) et pour aspirer le ciment (C) ainsi formé depuis ledit premier récipient (2) dans ledit deuxième récipient (3), ledit dispositif étant ***caractérisé en ce que*** ledit premier récipient (2) comprend de plus des moyens agitateurs (18) qui sont agencés pour se déplacer à l'intérieur de celui-ci de manière à mélanger ledit premier (A) et ledit deuxième (B) composants quand on agite ledit premier récipient (2).

2. Dispositif selon la revendication 1, ***caractérisé en ce que*** ledit deuxième composant (B) est à l'état de fluide, de pâte ou de gel, et ***en ce que*** ledit deuxième récipient (3) est une seringue ou un pistolet.

3. Dispositif selon l'une ou plusieurs des revendications précédentes, ***caractérisé en ce que*** ledit premier composant (A) se présente sous forme de poudre.

4. Dispositif selon l'une ou plusieurs des revendications précédentes, ***caractérisé en ce* qu'**il comprend des moyens de liaison temporaire (15, 16, 17) entre ladite ouverture (5) et ladite sortie (11).

5. Dispositif selon la revendication 4, ***caractérisé en ce que*** lesdits moyens de liaison temporaire (15) comprennent un connecteur mâle (16) et un connecteur femelle (17) correspondant de type Luer-Lok, formés respectivement au niveau de ladite sortie (11) et au niveau de ladite ouverture (5), ou vice versa.

6. Dispositif selon l'une ou plusieurs des revendications précédentes, ***caractérisé en ce que*** lesdits moyens agitateurs comprennent une bille (18) qui est insérée librement dans ledit premier récipient (2), la masse volumique de ladite bille (16) étant supérieure à celle dudit premier composant (A) et dudit ciment (C).

7. Dispositif selon l'une ou plusieurs des revendications précédentes, ***caractérisé en ce que*** ledit premier récipient (2) comprend des moyens (20) pour retenir lesdits moyens agitateurs (18) à une distance prédéterminée de ladite ouverture (5) afin d'empêcher son blocage.

8. Dispositif selon la revendication 7, ***caractérisé en ce que*** lesdits moyens de retenue (20) comprennent au moins une saillie qui est formée de manière à faire saillie de manière centripète près de ladite ouverture (5)

9. Dispositif selon la revendication 7, ***caractérisé en ce que*** lesdits moyens de retenue comprennent une pluralité d'ailes ou membrures (20) qui sont longitudinales, radiales et centripètes et qui sont distribuées le long du périmètre intérieur dudit premier récipient (2).

10. Dispositif selon l'une ou plusieurs des revendications précédentes, ***caractérisé en ce que*** ledit premier récipient (2) comprend une ouverture de remplissage (7) qui est associée à un élément de fermeture (8).

11. Dispositif selon la revendication 10, ***caractérisé en ce que*** ledit élément de fermeture est constitué d'un capuchon (8), des moyens étant prévus pour le couplage à verrouillage réciproque (9) dudit capuchon (8) avec ledit premier récipient (2).

12. Dispositif selon la revendication 11, ***caractérisé en ce que*** lesdits moyens (9) de couplage à verrouillage réciproque comprennent au moins une dent (26) qui est formée le long du périmètre extérieur dudit capuchon (8) et peuvent engager par encliquetage le bord de ladite ouverture de remplissage (7).

13. Dispositif selon la revendication 12, ***caractérisé en ce que*** les dents (26) sont disposées selon une configuration en arc le long dudit périmètre extérieur du capuchon (8), le long duquel elles sont réparties avec un espacement constant.

14. Dispositif selon l'une ou plusieurs des revendications 12 à 13, ***caractérisé en ce que*** ledit capuchon (8) comprend deux lèvres annulaires (22, 23) qui sont sensiblement coaxiales et repliées, sont formées à proximité dudit périmètre extérieur, et entre lesquelles est formée une bague annulaire (24), la bordure de ladite ouverture de remplissage (7) pouvant s'insérer dans ladite bague annulaire (24).

15. Dispositif selon la revendication 14, ***caractérisé en ce que*** lesdites dents (26) sont formées le long d'au moins l'une desdites lèvres et font face à l'intérieur de ladite bague annulaire (24).

16. Dispositif selon l'une ou plusieurs des revendications 10 à 15, ***caractérisé en ce que*** ledit élément de fermeture (8) est sensiblement en forme de dôme (8).

17. Dispositif selon l'une ou plusieurs des revendications précédentes, ***caractérisé en ce que*** lesdits premiers (6) ou deuxièmes (12) moyens de fermeture temporaire comprennent une butée.

18. Dispositif selon l'une ou plusieurs des revendications 1 à 16, ***caractérisé en ce que*** lesdits premiers (6) ou deuxièmes (12) moyens de fermeture temporaire comprennent une membrane ou un film du type déchirable, amovible ou autre.

19. Dispositif selon l'une ou plusieurs des revendications 1 à 16, ***caractérisé en ce que*** lesdits premiers (6) ou deuxièmes (12) moyens de fermeture temporaire comprennent un tampon qui est associé respectivement à ladite ouverture (5) ou à ladite sortie (11) le long de lignes qui sont au moins partiellement du type pré-affaibli.

20. Dispositif selon l'une ou plusieurs des revendications 1 à 16, ***caractérisé en ce que*** lesdits premiers (6) ou deuxièmes (12) moyens de fermeture temporaire comprennent un clapet anti-retour du type à bille.
